# EUROPEAN PATENT SPECIFICATION

(11) **EP 1 680 170 B1**
(45) Date of publication and mention of the grant of the patent: **25.01.2012**
(21) Application number: 04790467.7
(22) Date of filing: 14.10.2004
(51) Int. Cl.: A61F 2/84, A61M 25/00

(54) **CATHETER SYSTEM FOR STENTING BIFURCATED VESSELS**
KATHETERSYSTEM ZUR BEHANDLUNG VON GEGABELTEN GEFÄSSEN MIT STENTS
SYSTEME DE CATHETER POUR POSE D'UN STENT DANS DES VAISSEAUX BIFURQUES

(30) Priority: 16.10.2003 US 512259 P; 05.01.2004 US 534469 P; 27.04.2004 US 833494
(43) Date of publication of application: 19.07.2006
(73) Proprietor: MINVASYS, 92230 Gennevilliers (FR)
(72) Inventor: HILAIRE, Pierre, F-78160 MARLY LE ROI (FR); LEARY, James, GRANITE BAY, California 95746 (US); VAN DER LEEST, Machiel, F-75014 PARIS (FR)
(74) Representative: Hubert, Philippe
(86) International application number: PCT/EP2004/011622
(87) International publication number: WO 2005/039681

(56) References cited:
- WO-A-99/47202
- WO-A2-03/105922
- WO-A2-03/105922
- FR-A- 1 579 500
- US-A- 4 016 884
- US-A- 6 099 497
- US-A1- 2002 032 432
- US-A1- 2003 093 109

## Description

### FIELD OF THE INVENTION

The present invention relates generally to catheters and catheter systems for performing angioplasty and vascular stenting. More particularly it relates to a catheter system and method for stenting a vessel at a bifurcation or sidebranch of the vessel.

### BACKGROUND OF THE INVENTION

The following patents and patent applications relate to catheters and catheter systems for performing angioplasty and stenting of bifurcated vessels.

US 6,579,312 Stent and catheter assembly and method for treating bifurcations

US 6,540,779 Bifurcated stent with improved side branch aperture and method of making same

US 6,520,988 Endolumenal prosthesis and method of use in bifurcation regions of body lumens

US 6,508,836 Stent and catheter assembly and method for treating bifurcations

US 6,494,875 Bifurcated catheter assembly

US 6,475,208 Bifurcated catheter assembly

US 6,428,567 Stent and catheter assembly and method for treating bifurcations

US 6,387,120 Stent and catheter assembly and method for treating bifurcations

US 6,383,213 Stent and catheter assembly and method for treating bifurcations

US 6,371,978 Bifurcated stent delivery system having retractable sheath

US 6,361,544 Stent and catheter assembly and method for treating bifurcations

US 6,325,826 Extendible stent apparatus

US 6,264,682 Bifurcated stent delivery system having retractable sheath

US 6,258,073 Bifurcated catheter assembly

US 6,254,593 Bifurcated stent delivery system having retractable sheath

US 6,221,098 Stent and catheter assembly and method for treating bifurcations

US 6,210,380 Bifurcated catheter assembly

US 6,165,195 Stent and catheter assembly and method for treating bifurcations

US 6,142,973 Y-shaped catheter

US 6,117,117 Bifurcated catheter assembly

US 6,086,611 Bifurcated stent

US 5,720,735 Bifurcated endovascular catheter

US 5,669,924 Y-shuttle stent assembly for bifurcating vessels and method of using the same

US 5,613,980 Bifurcated catheter system and method

US 6,013,054 Multifurcated balloon catheter

US 4,896,670 Kissing balloon catheter

US 5,395,352 Y-adaptor manifold with pinch valve for an intravascular catheter

US 6,129,738 Method and apparatus for treating stenoses at bifurcated regions

US 6,544,219 Catheter for placement of therapeutic devices at the ostium of a bifurcation of a body lumen

US 6,494,905 Balloon catheter

US 5,749,825 Means method for treatment of stenosed arterial bifurcations

US 5,320,605 Multi-wire multi-balloon catheter

US 6,099,497 Dilatation and stent delivery system for bifurcation lesions

US 5,720,735 Bifurcated endovascular catheter

US 5,906,640 Bifurcated stent and method for the manufacture and delivery of same

US 5,893,887 Stent for positioning at junction of bifurcated blood vessel and method of making

US 5,755,771 Expandable stent and method of delivery of same

US 20030097169A1 Bifurcated stent and delivery system

US 20030028233A1 Catheter with attached flexible side sheath

US 20020183763A1 Stent and catheter assembly and method for treating bifurcations

US 20020156516A1 Method for employing an extendible stent apparatus

US 20020116047A1 Extendible stent apparatus and method for deploying the same

US 20020055732A1 Catheter assembly and method for positioning the same at a bifurcated vessel

WO 9944539A2 Dilatation and stent delivery system for bifurcation lesions

WO 03053507 Branched balloon catheter assembly

WO 9924104 Balloon catheter for repairing bifurcated vessels

WO 0027307 The sheet expandable trousers stent and device for its implantation

FR 2733689 Endoprosthesis with installation device for treatment of blood-vessel bifurcation stenosis.

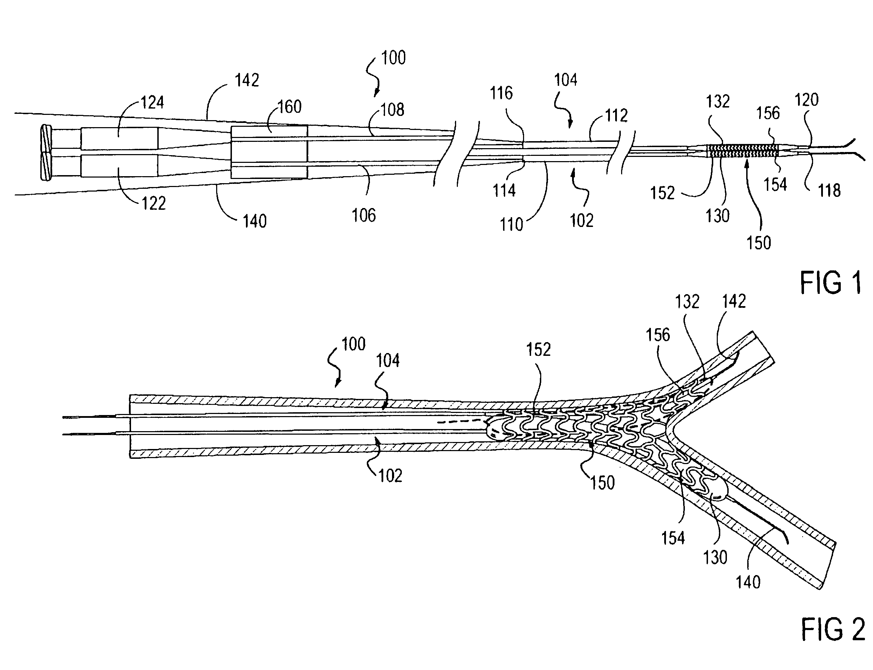

Document US 6 099 497 describes a system for the treatment of a lesion in a bifurcated vessel which may comprise two-ballon catheters and at least one stent, each catheter being able to be introduced into one of the branches of the bifurcated vessel when so directed by a guide wire figures 2A, 2B , 4-6).

The balloons may have a larger diameter in their distal regions as compared to their proximal regions, in such a way that the said distal regions push one another away and form an angle between them when the balloons are inflated (figure 2A).

WO 03/105922 discloses a catheter system comprising a guide (1000) intended to maintain a guide wire 204 and a first catheter 206 in proximity so as to prevent inadvertent forces against the channel 302 of a stent 300 mounted on the first catheter 206. A second balloon catheter 216 may be brought over the guide wire 204 through an opening 214 in the first stent 210 at a time when the guise 1000 wire has been withdrawn.

### SUMMARY OF THE INVENTION

The present invention relates generally to catheters and catheter systems for use in surgical and/or diagnostic procedures in which two catheters must be introduced into a patient's body, and wherein it is essential for the physician, methods of the invention can also be used for other applications as well in other fields of surgery and/or diagnosis.

While there has been considerable progress in methods of treating bifurcation lesions with stents in recent times, a need remains in the art to provide an improved catheter system which is easy to use for the physician and which enables efficient and accurate stent deployment and dilatation of the lesions that are to be treated.

In a first aspect, the invention is directed to a catheter system comprising:
a first catheter having a shaft with a proximal end and a distal end;
a second catheter having a shaft with a proximal end and a distal end; and
a linking device attachable near the proximal ends of the catheters for releasably linking the first catheter and the second catheter together in a side-by-side configuration and with the first catheter and the second catheter aligned with one another along a longitudinal axis.

The originality of the catheter system of the invention resides in the fact that it comprises a linking device allowing the two catheters to be advanced together in a side-by-side configuration as a unit by the physician until they are in the vicinity of the site to be treated, the linking device also enabling both catheters to be released when desired and maneuvered separately from the other components of the catheter system.

According to a preferred embodiment, the catheter system includes a first balloon catheter and a second balloon catheter, the above-mentioned linking device enabling the first and second balloon catheters to be held in a side-by-side configuration and aligned with one another along a longitudinal axis. It is however to be noted, and will be immediately evident to one skilled in the art, that the principles of the invention can also be applied to catheters other than balloon catheters.

The catheter system may include one or more vascular stents of various configurations mounted on the first and/or second balloon catheters. Hence, in one preferred embodiment of the present invention, a single vascular stent is mounted on either the first or the second balloon catheter. In a separate preferred embodiment, two vascular stents are provided, one of which is mounted on each of the balloon catheters. In a further preferred embodiment, a single vascular stent is provided, this stent being mounted simultaneously on both of the balloon catheters. In yet a further preferred embodiment, two vascular stents are provided, one of which is mounted simultaneously on both of the balloon catheters, and the other of which is mounted on just one of the balloon catheters. In all these embodiments, the linking device allows the catheter system to be advanced as a unit and helps prevent premature or inadvertent dislodgement of the stent or stents from the catheters.

Typically, the catheter system will also include a first and second steerable guidewire for guiding the first and second balloon catheters within the patient's blood vessels. Optionally, the linking device may also be configured to hold one or both of the guidewires stationary with respect to the catheter system.

In one embodiment of the invention, the first catheter and the second catheter are configured as rapid exchange balloon dilatation catheters.

Advantageously, in this latter case, a proximal section of each rapid exchange balloon dilatation catheter may be constructed of hypodermic tubing joined to a flexible distal section. Also in this latter case, at least one of the catheters may comprise an elongated flexible extension tube extending distally from the dilatation balloon.

In another embodiment of the invention, the first catheter and the second catheter are configured as over-the-wire catheter balloon dilatation catheters.

The catheter system may be arranged with the inflatable balloons in a side-by-side configuration for stenting the bifurcated vessels using a method similar to the "kissing balloons" technique. Alternatively, the catheter system may be arranged with the inflatable balloons in a low-profile staggered or tandem configuration for stenting the bifurcated vessels using a modified "kissing balloons" technique. When arranged in the staggered or tandem configuration, the second balloon catheter may optionally be constructed with a flexible tubular extension that extends the guidewire lumen distally from the inflatable balloon.

In a preferred embodiment the first catheter has an inflatable balloon mounted near the distal end of the first catheter and the second catheter has an inflatable balloon mounted near the distal end of the second catheter and the linking device links the first catheter and the second catheter together with the inflatable balloons arranged in a side-by-side configuration.

In a further preferred embodiment the first catheter has an inflatable balloon mounted near the distal end of the first catheter and the second catheter has an inflatable balloon mounted near the distal end of the second catheter and wherein the linking device links the first catheter and the second catheter together with the inflatable balloons arranged in a staggered or tandem configuration.

The linking device for use in the catheter system according to the first aspect of the present invention may have various configurations.

In a preferred embodiment of the first aspect of the present invention, the linking device of the catheter system comprises a body with a first channel and a second channel arranged in a side-by-side configuration, the first channel being configured to releasably hold the shaft of the first catheter and the second channel being configured to releasably hold the shaft of the second catheter.

In a further preferred embodiment of the first aspect of the present invention, the linking device of the catheter system comprises a body with a first channel and a second channel arranged in a side-by-side configuration, a first locking device associated with the first channel configured to releasably hold the shaft of the first catheter, and a second locking device associated with the second channel configured to releasably hold the shaft of the second catheter.

In a further preferred embodiment of the first aspect of the present invention, the linking device of the catheter system comprises a first linking member attached to the shaft of the first catheter and a second linking member attached to the shaft of the second catheter, the first linking member and the second linking member have interlocking features so that the first linking member and the second linking member can be releasably attached to one another.

In a further preferred embodiment of the first aspect of the present invention, the linking device of the catheter system comprises a peel-away sheath releasably attaching the shaft of the first catheter and the shaft of the second catheter together.

In a further preferred embodiment of the first aspect of the present invention, the linking device of the catheter system comprises a split-tube releasably attaching the shaft of the first catheter and the shaft of the second catheter together.

According now to a second aspect, the present invention is directed per se to a linking device as described above in relation to the first aspect of the present invention.

The originality of the linking device according to the second aspect of the invention resides in the fact that it is configured so that one or both of the (e.g. balloon) catheters and/or the guidewires can be released from the linking device and maneuvered separately from the rest of the catheter system.

In one embodiment the linking device is self-releasing in the sense that the linking device demounts itself from the first and second (e.g. balloon) catheters as the catheter system is advanced into the patient's body. On account of the fact that the linking device is attachable near the proximal ends of the catheters, it may be released by the physician in a very convenient manner, thereby allowing the to catheters to be maneuvered separately.

In a preferred embodiment according to the second aspect of the present invention, the catheter linking device comprises: a linking device body having a first channel and a second channel arranged in a side-by-side configuration within the linking device body, the first channel being configured to releasably hold a shaft of a first catheter and a second channel being configured to releasably hold a shaft of a second catheter.

In another preferred embodiment according to the second aspect of the present invention, the catheter linking device further comprises a first locking device associated with the first channel configured to releasably hold the shaft of the first catheter, and a second locking device associated with the second channel configured to releasably hold the shaft of the second catheter.

In another preferred embodiment according to the second aspect of the present invention, the catheter linking device comprises a split-tube releasably attaching the shaft of the first catheter and the shaft of the second catheter together.

### BRIEF DESCRIPTION OF THE DRAWINGS

FIG 1 shows a first embodiment of a catheter system for stenting bifurcated vessels according to the present invention.
FIG 2 shows the catheter system of FIG 1 in use for stenting a bifurcated vessel with a bifurcated stent.
FIG 3 shows a variation of the catheter system of FIG 1 for stenting a bifurcated vessel.
FIG 4 shows the catheter system of FIG 3 in use for stenting a bifurcated vessel.
FIG 5 shows a second embodiment of a catheter system for stenting bifurcated vessels.
FIGS 6A-9 show various embodiments of a linking device for use with the catheter system of the present invention.
FIGS 10-13 show the catheter system of FIG 5 in use for stenting a bifurcated vessel using a main stent and a sidebranch stent.
FIG 14 shows a third embodiment of a catheter system for stenting bifurcated vessels.
FIG 15 shows a cross section of a split-tube linking device for the catheter system of FIG 14.
FIG 16 shows an alternate cross section of a split-tube linking device for the catheter system of FIG 14.
FIG 17 shows the catheter system of FIG 14 in use.
FIG 18 shows a distal portion of a catheter system for stenting bifurcated vessels.
FIG 19 shows a bifurcated vessel after stenting with the catheter system of FIG 18.

### DETAILED DESCRIPTION OF THE INVENTION

FIG 1 shows a first embodiment of the catheter system 100 of the present invention for stenting bifurcated vessels. The catheter system 100 includes a first balloon catheter 102 and a second balloon catheter 104. An inflatable balloon 130, 132 is mounted on each of the first and second balloon catheters 102, 104 near the distal end of the catheters. A balloon-expandable vascular stent 150 is mounted on the catheter system 100, typically by crimping or swaging the stent 150 over both of the inflatable balloons 130, 132. The stent structure is shown generically and is not intended to be limited to any particular strut geometry. Typically, the catheter system 100 will also include a first and second steerable guidewire 140, 142 for guiding the first and second balloon catheters 102, 104 within the patient's blood vessels. The first and second steerable guidewires 140, 142 will typically have a diameter of 0.010-0.018 inches (approximately 0.25-0.46 mm), preferably 0.014 inches (approximately 0.36 mm). A linking device 160 releasably joins the first balloon catheter 102 and the second balloon catheter 104 together near the proximal ends of the catheters. The linking device 160 holds the first and second balloon catheters 102, 104 in a side-by-side configuration and aligned with one another along a longitudinal axis. The linking device 160 allows the catheter system 100 to be advanced as a unit and helps prevent premature or inadvertent dislodgement of the stent 150 from the catheters. Optionally, the linking device 160 may also be configured to hold one or both of the guidewires 140, 142 stationary with respect to the catheter system 100.

The first and second balloon catheters 102, 104 may be of any known construction for balloon angioplasty or stent delivery catheters, including rapid exchange and over-the-wire catheter constructions. In a particularly preferred embodiment, the first and second balloon catheters are constructed as rapid exchange catheters, wherein a proximal section 106, 108 of each catheter is constructed of hypodermic tubing, which may be formed from stainless steel, a superelastic nickel-titanium or titanium-molybdenum alloy or the like. The exterior of the proximal section 106, 108 is preferably coated with PTFE or another highly lubricious coating. A proximal connector 122, 124, such as a luer lock connector or the like, is attached at the proximal end of the proximal section 106, 108 and communicates with a balloon inflation lumen that extends through the hypodermic tubing. Each catheter includes a flexible distal section 110, 112 joined to the proximal section 106, 108. Typically, the flexible distal section 110, 112 has two lumens that extend through most of its length, including a guidewire lumen that extends from a proximal guidewire port 114, 116 to a distal port 118, 120 at the distal end of the catheter, and a balloon inflation lumen that connects from the balloon inflation lumen of the proximal section 106, 108 to the interior of the inflatable balloon 130, 132, which is mounted near the distal end of the flexible distal section 110, 112. The first and second inflatable balloons 130, 132 may have the same length and diameter and pressure compliance or they may have different lengths, diameters and/or pressure compliances, depending on the geometry of the target vessel that the catheter system 100 is intended for. The inflatable balloons 130, 132 may be made from a variety of known angioplasty balloon materials, including, but not limited to, PVC, polyethylene, polyolefin, polyamide, polyester, PET, PBT, and blends, alloys, copolymers and composites thereof. The first and second inflatable balloons 130, 132 may be made from the same material or different materials. The flexible distal section 110, 112 is typically constructed of flexible polymer tubing and may have a coaxial or multilumen construction. Preferably, one, two or more radiopaque markers are mounted on the flexible distal section 110, 112 to indicate the location of the inflatable balloons 130, 132 under fluoroscopic imaging. A transition element may be included to create a gradual transition in stiffness between the proximal section 106, 108 and the flexible distal section 110, 112, and to avoid a stress concentration at the juncture between the two sections. The transition element may be constructed as a tapered or spiral wound element that is formed as an extension of the hypodermic tubing or from a separate piece of wire or tubing.

In this illustrative example, the catheter system 100 is configured for delivering a Y-shaped bifurcated stent 150. The bifurcated stent 150 has a main trunk 152 connected to first and second sidebranches 154, 156 of the stent. The catheter system 100 is prepared for use by inserting the inflatable balloons 130, 132 in a deflated and folded state through the main trunk 152 of the bifurcated stent 150, with one balloon extending into each of the first and second sidebranches 154, 156. The bifurcated stent 150 is then crimped or swaged over the inflatable balloons 130, 132. A support wire may be inserted into each of the guidewire lumens to support them during the crimping or swaging step. The proximal sections 106, 108 of the catheters are inserted into the linking device 160 to hold the first and second balloon catheters 102, 104 in a side-by-side configuration and aligned with one another along a longitudinal axis. This preparation may be carried out at the manufacturing facility or it may be performed at the point of use by a medical practitioner.

FIG 2 shows the catheter system 100 of FIG 1 in use for stenting a bifurcated vessel. The catheter system 100 is inserted into a body lumen that is desired to be stented and advanced as a unit to the point of the bifurcation. For stenting coronary arteries or carotid arteries, the catheter system 100 is typically inserted through a guiding catheter that has been previously positioned at the ostium of the target vessel. For stenting in peripheral arteries or other body lumens, the catheter system 100 may be inserted directly into the vessel, for example using the Seldinger technique or an arterial cutdown, or it may be inserted through an introducer sheath or guiding catheter placed into the vessel. The first and second balloon catheters 102, 104 are maneuvered with the help of the steerable guidewires 140, 142 so that the first and second inflatable balloons 130, 132, with the first and second sidebranches 154, 156 of the stent 150 mounted thereon, extend into the respective first and second sidebranches of the bifurcated vessel. The first and second inflatable balloons 130, 132 are inflated separately and/or together to expand the stent 150 and to seat it securely within the vessel, as shown in FIG 2. This is similar to the "kissing balloons" technique that has been previously described in the literature. An advantage of the present invention over prior methods is that the linking device 160 allows the catheter system 100 to be advanced as a unit and helps prevent premature or inadvertent dislodgement of the stent 150 from the catheters.

Once the stent 150 has been deployed, both balloons 130, 132 are deflated and the catheter system 100 is withdrawn from the patient. Alternatively, one or both of the balloon catheters 102, 104 can be released from the linking device 160 and used separately for dilating and/or stenting other vessels upstream or downstream of the stent 150.

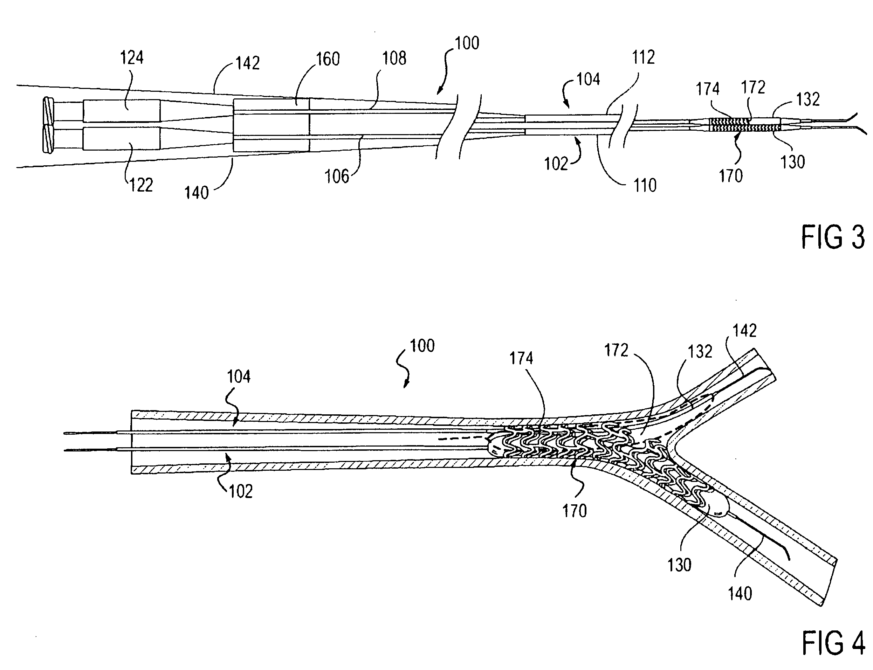

FIG 3 shows a variation of the catheter system 100 of the present invention for stenting a bifurcated vessel. The construction of the catheter system 100 is very similar to the catheter system described above in connection with FIG 1 with the exception that the system utilizes a straight, i.e. non-bifurcated, stent 170. The stent structure is shown generically and is not intended to be limited to any particular strut geometry. In one particularly preferred embodiment, the stent 170 is in the form of an open-cell stent, having a cylindrical body 174 with one or more side openings 172 that are suitable for placement at a bifurcation or sidebranch of the vessel without hindering blood flow into the sidebranch. Because of their flexibility and open structure, open-cell stents are well suited for stenting bifurcated vessels. The side openings 172 can be expanded or remodeled with a dilatation balloon inserted through the side opening or with two dilatation balloons, using the "kissing balloons" technique. A closed-cell stent with large side openings and/or expandable side openings may also be utilized. Alternatively, the catheter system may utilize a side-hole stent intended for stenting bifurcations or for stenting a main vessel at the location of a sidebranch vessel. In this case, the stent has an approximately cylindrical body with a side hole intended to be positioned at the site of a sidebranch vessel. The side hole may be preformed in the stent or it may be a slit or a potential hole that can be expanded to form a side hole.

The catheter system 100 is prepared for use by inserting the inflatable balloons 130, 132 in a deflated and folded state into the stent 170, with the first balloon 130 extending all the way through the cylindrical body 174 and the second balloon 132 exiting the cylindrical body 174 at the side opening 172 that is intended to be positioned at the bifurcation or sidebranch vessel. Alternatively, the second balloon 132 may be positioned proximal to the side opening 172 so that only the distal tip of the catheter 104 or only the guidewire 142 exits the cylindrical body 174 at the side opening 172 to decrease the distal crossing profile of the catheter system 100. The stent 170 is then crimped or swaged over the inflatable balloons 130, 132. A support wire may be inserted into each of the guidewire lumens to support them during the crimping or swaging step. The proximal sections 106, 108 of the catheters are inserted into the linking device 160 to hold the first and second balloon catheters 102, 104 in a side-by-side configuration and aligned with one another along a longitudinal axis. This preparation may be carried out at the manufacturing facility or it may be performed at the point of use by a medical practitioner.

FIG 4 shows the catheter system 100 of FIG 3 in use for stenting a bifurcated vessel. The catheter system 100 is inserted into a body lumen that is desired to be stented and advanced to the point of the bifurcation. For stenting coronary arteries or carotid arteries, the catheter system 100 is typically inserted through a guiding catheter that has been previously positioned at the ostium of the target vessel. For stenting in peripheral arteries or other body lumens, the catheter system 100 may be inserted directly into the vessel, for example using the Seldinger technique or an arterial cutdown, or it may be inserted through an introducer sheath or guiding catheter placed into the vessel. The first and second balloon catheters 102, 104 are maneuvered with the help of the steerable guidewires 140, 142 so that the first and second inflatable balloons 130, 132, with the stent mounted thereon, extend into the respective first and second sidebranches of the bifurcated vessel. The first inflatable balloon 130 will typically be positioned in the larger of the two sidebranches or in the main lumen of the vessel at the location of a smaller sidebranch vessel. The first inflatable balloon 130 is inflated to expand the stent and to seat it securely within the vessel, as shown in FIG 4. Then, the first inflatable balloon 130 is deflated and the second inflatable balloon 132 is inflated to expand the side opening 172 at the location of the second sidebranch vessel. Optionally, the first and second inflatable balloons 130, 132 may be inflated simultaneously using the "kissing balloons" technique.

Once the stent 170 has been deployed, both balloons 130, 132 are deflated and the catheter system 100 is withdrawn from the patient. Alternatively, one or both of the balloon catheters 102, 104 can be released from the linking device 160 and used separately for dilating and/or stenting other vessels upstream or downstream of the stent 170. Optionally, a sidebranch stent may be placed in the second sidebranch vessel before or after deployment of the stent 170.

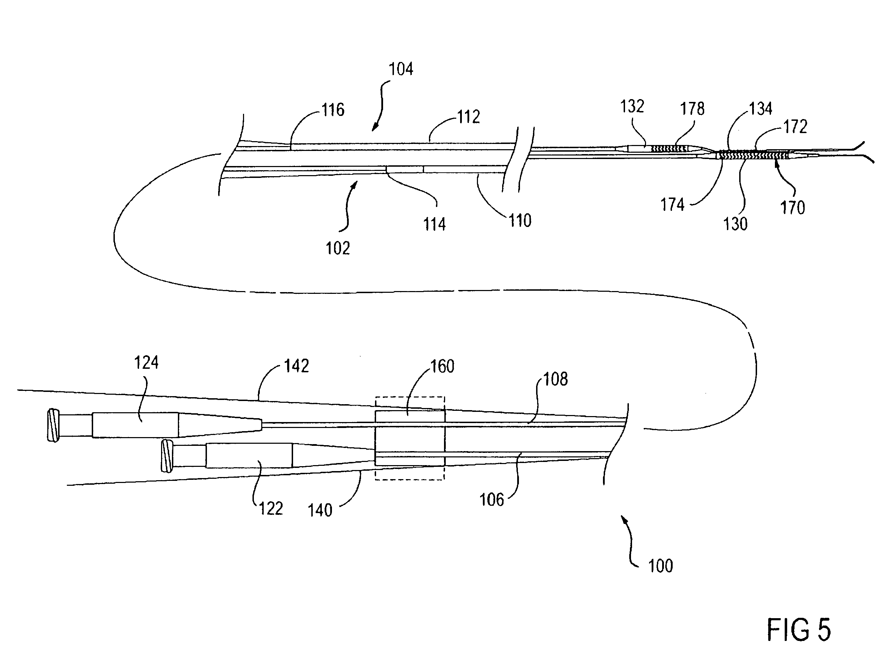

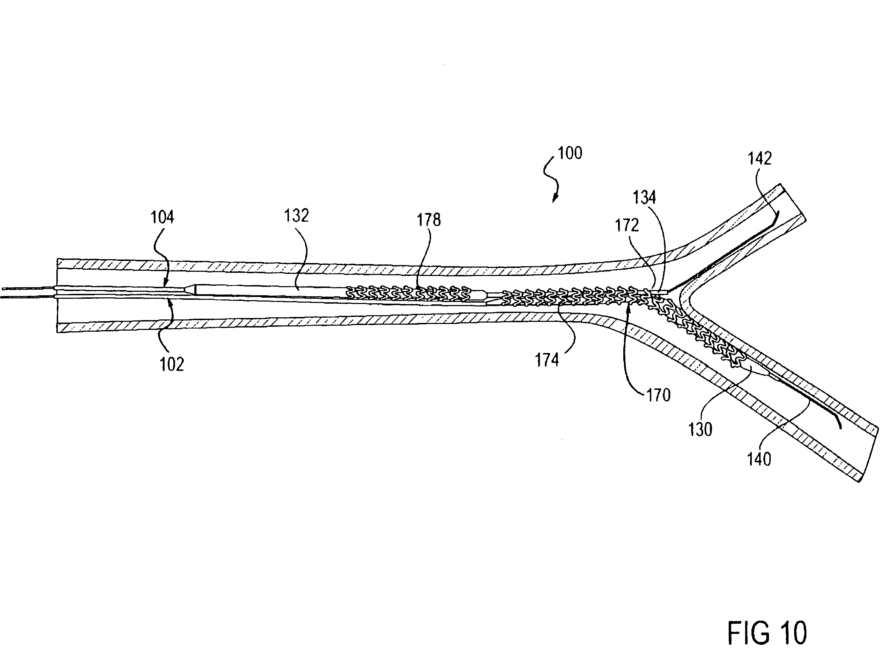

FIG 5 shows a second embodiment of the catheter system 100 for stenting bifurcated vessels. The construction of the catheter system 100 is very similar to the catheter system described above in connection with FIGS 1 and 3, with the exception that the second balloon catheter 104 is constructed with a flexible tubular extension 134 connected to the distal end of the catheter. The guidewire lumen extends through the flexible tubular extension 134. The flexible tubular extension 134 allows the first and second inflatable balloons 130, 132 to be assembled together in a staggered or tandem initial position. This variation of the catheter system 100 utilizes a main stent 170, which is typically a straight, i.e. non-bifurcated, stent, as described above. In addition, the catheter system 100 may optionally utilize a sidebranch stent 178. The stent structures are shown generically and are not intended to be limited to any particular strut geometry. These distal features of the catheter system 100 can be seen in greater detail in the enlarged view of FIG 10.

The catheter system 100 is prepared for use by first inserting the second inflatable balloon 132 in a deflated and folded state through the optional sidebranch stent 178 and crimping or swaging the sidebranch stent 178 over the second inflatable balloon 132. Alternatively, the sidebranch stent 178 may be mounted on a separate balloon catheter for use with the catheter system 100. The first inflatable balloon 130 is then inserted in a deflated and folded state into the main stent 170, with the first balloon 130 extending all the way through the cylindrical body 174. The flexible tubular extension 134 of the second balloon catheter 104 is inserted into the main stent 170 alongside the first balloon 130 with the flexible tubular extension 134 exiting the cylindrical body 174 at the side opening 172 that is intended to be positioned at the bifurcation or sidebranch vessel. Preferably, the flexible tubular extension 134 terminates at the side opening 172 of the main stent 170 to reduce the crossing profile of the distal portion of the stent 170. Alternatively, the flexible tubular extension 134 may extend distally from the side opening 172 if desired. The main stent 170 is then crimped or swaged over the first inflatable balloon 130 and the flexible tubular extension 134. A support wire may be inserted into each of the guidewire lumens to support them during the crimping or swaging step. The proximal sections 106, 108 of the catheters are inserted into the linking device 160 to hold the first and second balloon catheters 102, 104 in a side-by-side configuration and in a desired alignment with one another along the longitudinal axis. This preparation may be carried out at the manufacturing facility or it may be performed at the point of use by a medical practitioner.

In an alternate embodiment of the catheter system 100 of FIG 5, the second balloon catheter 104 may be constructed without a flexible tubular extension 134. In this case, the distal tip of the second balloon catheter 104 would be positioned proximal to the main stent 170 and the second steerable guidewire 142 would be inserted into the main stent 170 alongside the first balloon 130 with the guidewire 142 exiting the cylindrical body 174 at the side opening 172. This would provide an even lower crossing profile for the catheter system 100.

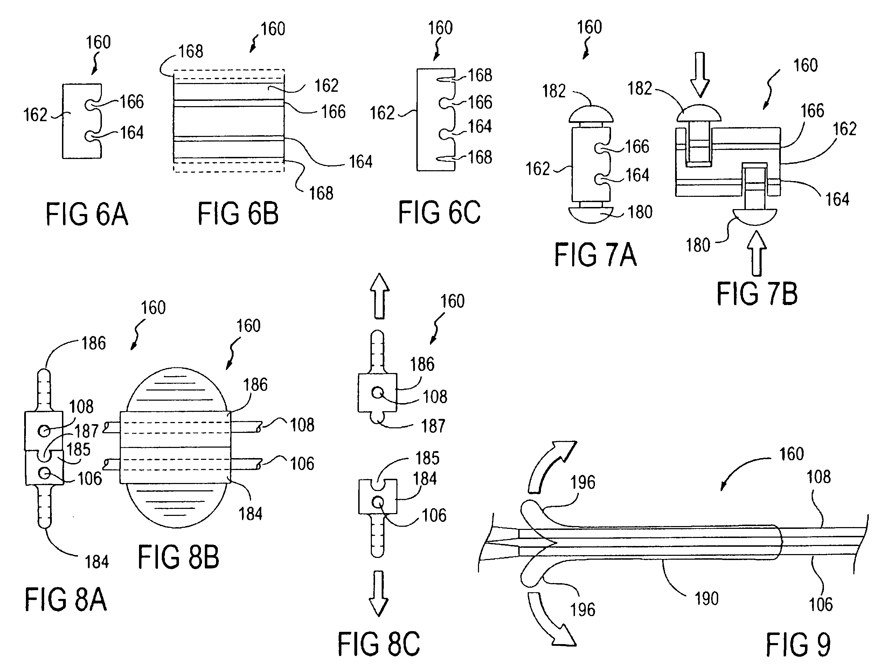

FIGS 6A-9 show various embodiments of a linking device 160 for use with the catheter system 100 of the present invention.

FIG 6A shows an end view and 6B shows a front view of a first embodiment of a linking device 160. The linking device 160 has a body 162 with a first channel 164 and a second channel 166 extending along a surface of the body in a side-by-side configuration, preferably with the first and second channels 164, 166 approximately parallel to one another. The first and second channels 164, 166 are preferably undercut and sized to have a captive interference fit with the proximal sections 106, 108 of the first and second balloon catheters 102, 104. The linking device 160 is preferably molded of a flexible polymer or elastomer with a high coefficient of friction so that it effectively grips the proximal sections 106, 108 of the first and second balloon catheters 102, 104 when they are inserted into the first and second channels 164, 166. In use, the linking device 160 holds the first and second balloon catheters 102, 104 arranged in a side-by-side configuration and aligned with one another along a longitudinal axis. The linking device 160 allows the catheter system 100 to be advanced as a unit and helps prevent premature or inadvertent dislodgement of the stent from the catheters. When it is desired, one or both of the balloon catheters 102, 104 can be released from the linking device 160 and maneuvered separately from the rest of the catheter system 100.

Optionally, the linking device 160 of FIG 6B may also be configured to hold one or both of the guidewires 140, 142 stationary with respect to the catheter system 100. In this case, the body 162 of the linking device 160 would include one or two slots 168, shown in dashed lines in FIG 6B, that are sized and configured to create a captive interference fit with the proximal section of the guidewires 140, 142. FIG 6C shows an end view of the linking device 160 with optional slots 168 for holding the guidewires 140, 142. When it is desired, the guidewires 140, 142 can be released from the linking device 160 and maneuvered separately from the rest of the catheter system 100.

In an alternative embodiment, the linking device 160 of FIGS 6A-6B may be permanently attached to one of the balloon catheters and releasably attached to the other. In another alternative embodiment, the linking device 160 may be configured to attach instead to the proximal connectors 122, 124 of the balloon catheters 102, 104 or it may be molded into the proximal connectors 122, 124.

FIG 7A shows an end view and 7B shows a front view of a second embodiment of the linking device 160. The linking device 160 has a body 162 with a first channel 164 and a second channel 166 extending along one surface of the body in a side-by-side configuration, preferably with the first and second channels 164, 166 approximately parallel to one another. The first and second channels 164, 166 are preferably undercut and sized to have a captive sliding fit with the proximal sections 106, 108 of the first and second balloon catheters 102, 104. A first locking device 180 is associated with the first channel 164, and a second locking device 182 is associated with the second channel 166. The first and second locking devices 180, 182 are configured to releasably lock the proximal sections 106, 108 of the first and second balloon catheters 102, 104 in a desired alignment with one another along the longitudinal axis. Each of the locking devices 180, 182 will typically include a spring or other biasing member to hold the locking device in a locked position and a push button or other actuating member to release the locking device. The linking device 160 allows the catheter system 100 to be advanced as a unit and helps prevent premature or inadvertent dislodgement of the stent from the catheters. When it is desired, one or both of the locking devices 180, 182 can be released to allow one of the balloon catheters 102, 104 to be advanced or retracted with respect to the other to adjust their longitudinal alignment. In addition, one or both of the balloon catheters 102, 104 can be released completely from the linking device 160 and maneuvered separately from the rest of the catheter system 100.

Optionally, the linking device 160 of FIGS 7A-7B may also be configured to hold one or both of the guidewires 140, 142 stationary with respect to the catheter system 100. In this case, the body 162 of the linking device 160 would include one or two additional locking devices, or slots or other structures configured to grip the proximal section of the guidewires 140, 142. When it is desired, the guidewires 140, 142 can be released from the linking device 160 and maneuvered separately from the rest of the catheter system 100.

In an alternative embodiment, the linking device 160 of FIGS 7A-7B may be permanently attached to one of the balloon catheters and releasably attached to the other.

FIG 8A shows an end view and 8B shows a front view of a third embodiment of the linking device 160. The linking device 160 has a first linking member 184 attached to the proximal section 106 of the first balloon catheter 102 and a second linking member 186 attached to the proximal section 108 of the second balloon catheter 104. The first linking member 184 and the second linking member 186 have interlocking features so that the two catheters can be releasably attached to one another. In the example shown, the interlocking features are corresponding male 187 and female 185 elements that can be attached and detached to one another in the manner of a snap or zip-lock device. FIG 8C shows an end view of the linking device 160 with the first linking member 184 and the second linking member 186 detached from one another. Optionally, the linking device 160 can be configured so that the balloon catheters 102, 104 can be attached to one another in different longitudinal alignments. In other embodiments, the linking device 160 of FIGS 8A-8C may utilize alternative interlocking features such as clamps, snaps, hook-and-loop fasteners, a releasable adhesive, a repositionable adhesive, etc.

Optionally, the linking device 160 of FIGS 8A-8C may also be configured to hold one or both of the guidewires 140, 142 stationary with respect to the catheter system 100. In this case, one or both of the linking members 184, 186 would include a locking device, slot or other structure configured to hold the proximal section of one of the guidewires 140, 142. This configuration would allow each guidewire and balloon catheter pair to be moved as a unit separately from the rest of the catheter system 100 when the linking members 184, 186 are separated. When it is desired, one or both of the guidewires 140, 142 can be released from the linking members 184, 186 and maneuvered separately from the rest of the catheter system 100.

FIG 9 shows a fourth embodiment of the linking device 160 that utilizes a peel-away sheath 190 for attaching the proximal sections 106, 108 of the first and second balloon catheters 102, 104 together. The peel-away sheath 190 may be made from heat shrink polymer tubing that is heat shrunk onto the proximal sections 106, 108 of the first and second balloon catheters 102, 104 to lock them together in a desired alignment with one another along the longitudinal axis. The peel-away sheath 190 has tabs or handles 196 to facilitate peeling the peel-away sheath 190 apart to release the balloon catheters 102, 104 so that they can be maneuvered separately from one another. The peel-away sheath 190 may utilize features, such as polymer orientation, perforations and/or an incised groove, to assure that the peel-away sheath 190 will peel apart along a longitudinal dividing line.

FIGS 10-13 show the catheter system 100 of FIG 5 in use for stenting a bifurcated vessel using a main stent 170 and a sidebranch stent 178. The catheter system 100 is inserted into a body lumen that is desired to be stented and advanced to the point of the bifurcation. For stenting coronary arteries or carotid arteries, the catheter system 100 is typically inserted through a guiding catheter that has been previously positioned at the ostium of the target vessel. For stenting in peripheral arteries or other body lumens, the catheter system 100 may be inserted directly into the vessel, for example using the Seldinger technique or an arterial cutdown, or it may be inserted through an introducer sheath or guiding catheter placed into the vessel. The staggered or tandem initial position of the first and second inflatable balloons 130, 132 provides a very low crossing profile. The low crossing profile allows the catheter system 100 with a 3.0 or 3.5 mm (expanded diameter) coronary stent 170 mounted on it to be delivered through a 6 French (approximately 2 mm external diameter) guiding catheter, which will typically have an internal diameter of 0.066-0.071 inches (approximately 1.68-1.80 mm internal diameter).

The catheter system 100 is maneuvered with the help of the steerable guidewires 140, 142 so that the first inflatable balloon 130, with the main stent 170 mounted on it, extends into the first sidebranch of the bifurcated vessel and the second steerable guidewire 142 extends into the second sidebranch, as shown in FIG 10. The first inflatable balloon 130 will typically be positioned in the larger of the two sidebranches or in the main lumen of the vessel at the location of a smaller sidebranch vessel.

When advancing the catheter system 100, the second steerable guidewire 142 may be positioned with its distal tip withdrawn into the flexible tubular extension 134 of the second balloon catheter 104 until the catheter system 100 reaches the bifurcation so that it will not be inadvertently damaged or interfere with advancement of the catheter system 100. This can be facilitated by inserting the proximal section of the second guidewire 142 into the optional slot or locking device 168 on the linking device 160. When the distal tip of the second balloon catheter 104 is in the vicinity of the sidebranch vessel, the second steerable guidewire 142 can be released from the linking device 160 and advanced with its distal tip extending from the flexible tubular extension 134 to engage the sidebranch vessel.

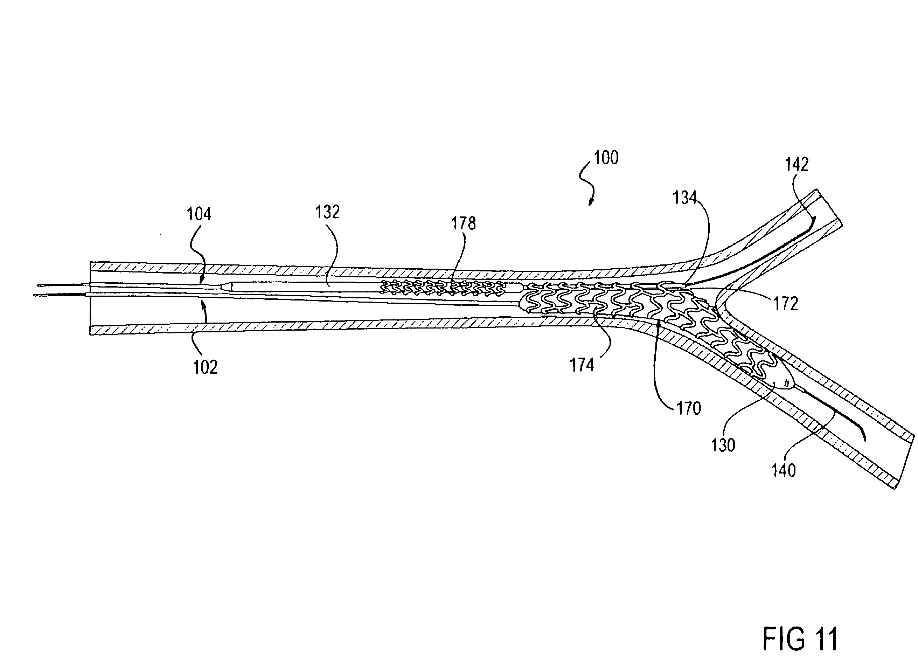

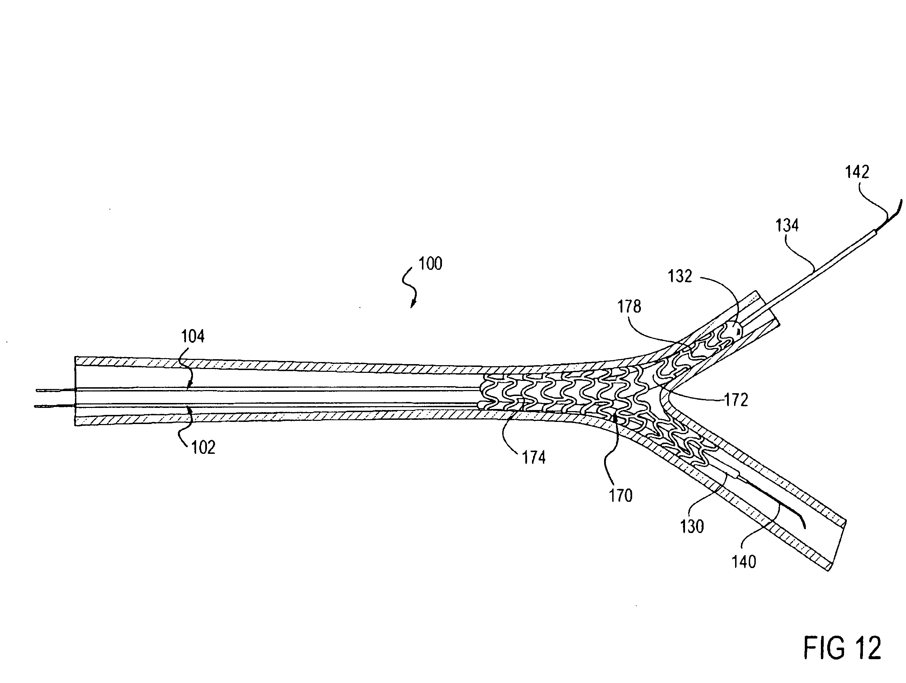

Once the main stent 170 is in the desired position, the first inflatable balloon 130 is inflated to expand the main stent 170 and to seat it securely within the vessel, as shown in FIG 11. Then, the first inflatable balloon 130 is deflated and the linking device 160 is released so that the second balloon catheter 104 can be advanced into the second sidebranch. The second inflatable balloon 132 is inflated to expand the sidebranch stent 178 and to seat it securely within the second sidebranch vessel, while simultaneously opening the side opening 172 in the main stent 170, as shown in FIG 12. Alternatively, if a sidebranch stent is not used or if it is to be delivered on a separate balloon catheter, the second inflatable balloon 132 is inflated to open the side opening 172 in the main stent 170 at the location of the second sidebranch vessel. Optionally, the first and second inflatable balloons 130, 132 may be inflated simultaneously using the "kissing balloons" technique.

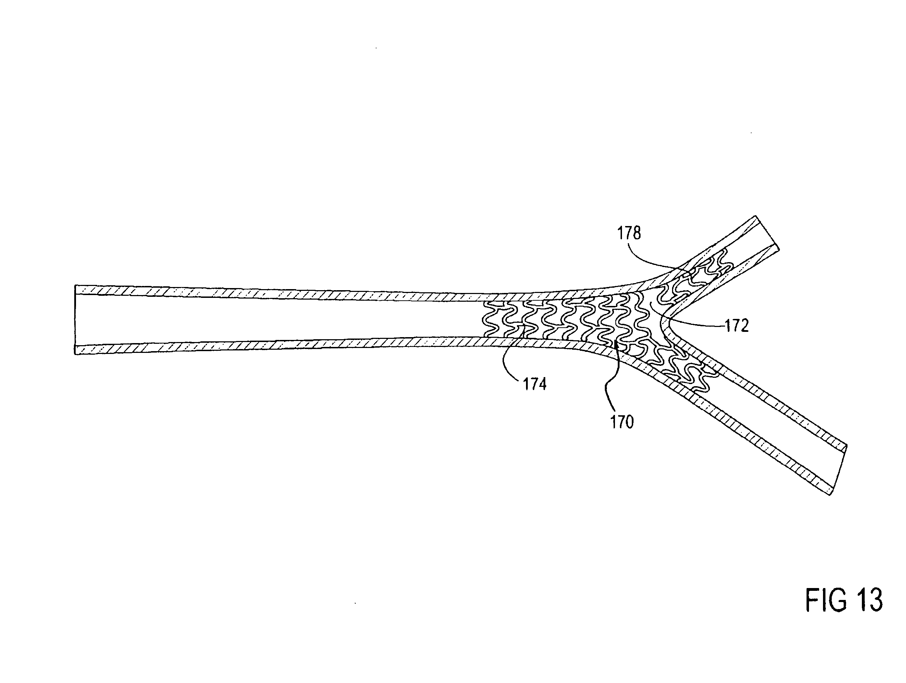

Once the stents 170, 180 have been deployed, both balloons 130, 132 are deflated and the catheter system 100 is withdrawn from the patient as shown in FIG 13. Alternatively, one or both of the balloon catheters 102, 104 can be released from the linking device 160 and used separately for dilating and/or stenting other vessels upstream or downstream of the main stent 170. Optionally, a sidebranch stent 178 may be placed in the second sidebranch vessel using a separate balloon catheter before or after deployment of the main stent 170.

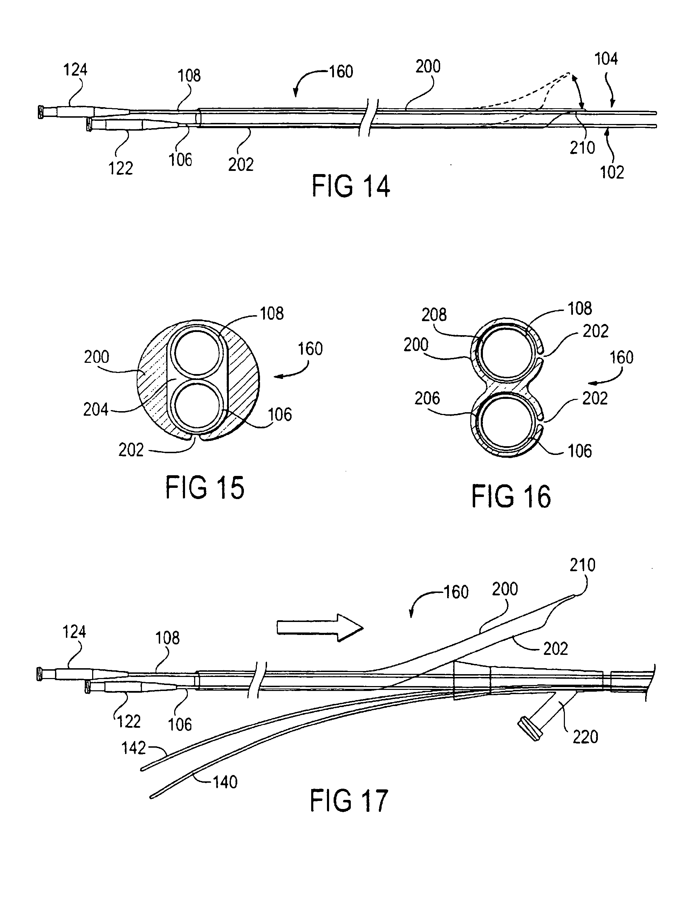

FIG 14 shows a third embodiment of a catheter system 100 for stenting bifurcated vessels utilizing a linking device 160 constructed of an elongated split-tube 200. The split-tube 200 of the linking device 160 is configured to hold the proximal sections 106, 108 of the first and second balloon catheters 102, 104 arranged in a side-by-side configuration and aligned with one another along a longitudinal axis. A longitudinal split 202 extends the length of the split-tube 200. The longitudinal split 202 allows the split-tube 200 to be placed over the proximal sections 106, 108 of the catheters 102, 104 during catheter preparation and to be removed from the catheters 102, 104 at the appropriate time during the stenting procedure. The length of the split-tube 200 can vary. Good results were obtained with a catheter system 100 having a split-tube 200 that extends along most of the proximal sections 106, 108 of the balloon catheters 102, 104 between the proximal hubs 122, 124 and the proximal guidewire ports 114, 116 of the rapid exchange catheters. Preferably, the split-tube 200 of the linking device 160 is configured with a distal pull-tab 210 or other feature to facilitate lifting the distal part of the split-tube 200 to remove the linking device 160 and release the balloon catheters 102, 104 so that they can be maneuvered separately from one another. The pull-tab 210 is preferably located on a side of the split-tube 200 opposite to the longitudinal split 202. The pull-tab 210 can be formed by skiving or cutting away part of the tube 200 as shown.

FIG 15 shows a cross section of one embodiment of the split-tube 200 of the linking device 160 for the catheter system 100 of FIG 14. The split-tube 200 has an inner lumen 204 that is sized and configured to hold the proximal sections 106, 108 of the first and second balloon catheters 102, 104 together with sufficient friction that the catheter system 100 can be advanced as a unit without any relative movement of the two catheters. In one particularly preferred embodiment, the split-tube 200 is manufactured as an extruded profile with an approximately circular outer profile and an approximately oval inner lumen 204. The longitudinal split 202 connects the inner lumen 204 with the exterior of the split-tube 200 at a thin part of the wall that coincides with the major axis of the oval inner lumen 204. The longitudinal split 202 is preferably formed during the extrusion of the split-tube 200. Alternatively, the tube 200 can be extruded without the longitudinal split 202 and then slitted along the length to form the longitudinal split 202 in a secondary operation. Suitable materials for the split-tube 200 include polyamide copolymers (e.g. PEBAX 6333 or PA 8020 from ATOFINA), polypropylene, and any extrudable medical grade polymer with a suitable combination of strength, flexibility and friction characteristics.

The split-tube 200 of the linking device 160 can be made with many other possible configurations, including single-lumen and multiple-lumen configurations, and may include one or more longitudinal splits 202. By way of example, FIG 16 shows an alternate cross section of a split-tube 200 of the linking device 160 for the catheter system 100 of FIG 14. In this embodiment, the split-tube 200 has a first inner lumen 206 that is sized and configured to hold the proximal section 106 of the first balloon catheter 102 and a second inner lumen 208 that is sized and configured to hold the proximal section 108 of the second balloon catheter 104. The inner lumens 206, 208 are sized and configured to hold the proximal sections 106, 108 of the first and second balloon catheters 102, 104 with sufficient friction that the catheter system 100 can be advanced as a unit without any relative movement of the two catheters. Two longitudinal splits 202 connect the inner lumens 206, 208 with the exterior of the split-tube 200. The two longitudinal splits 202 are preferably located on the same side of the split-tube 200 opposite to the distal pull-tab 210 to facilitate removal of the linking device 160 from both catheters 102, 104 simultaneously. The longitudinal splits 202 are preferably formed during the extrusion of the split-tube 200. Alternatively, the tube 200 can be extruded without the longitudinal splits 202 and then slitted along the length to form the longitudinal splits 202 in a secondary operation. Optionally, the linking device 160 in FIG 15 or FIG 16 can include additional lumens, slots or other structures to hold one or both of the guidewires 140, 142 stationary with respect to the catheter system 100.

FIG 17 shows the catheter system 100 of FIG 14 in use. The linking device 160 with the split-tube 200 has the advantage that, once it is started, the split-tube 200 will demount itself as the catheter system 100 is advanced so that the physician does not need to unpeel, remove or displace a linking member that would otherwise require a "third hand". The catheter system 100 is prepared for use by aligning the first and second balloon catheters 102, 104 in the desired longitudinal alignment and then pressing the longitudinal split 202 of the split-tube 200 against the proximal sections 106, 108 of the catheters until they are enclosed within the inner lumen 204 (or lumens 206, 208) of the split-tube 200, as shown in FIG 14. A stent or stents may then be crimped or mounted on the balloons 130, 132 in the desired configuration. This preparation may be carried out at the manufacturing facility or it may be performed at the point of use by a medical practitioner. The distal ends of the catheters 102, 104 with the stent or stents mounted thereon are inserted into the patient in the usual manner through a guiding catheter with a Y-fitting 220 or other hemostasis adapter on the proximal end of the guiding catheter. The distal pull-tab 210 is pulled toward the side to start demounting the split-tube 200 from the balloon catheters 102, 104, and then the first and second balloon catheters 102, 104 are advanced as a unit. As shown in FIG 17, when the split-tube 200 encounters the Y-fitting 220, the split-tube 200 will peel away or demount itself from the proximal sections 106, 108 of the balloon catheters 102, 104. The stent or stents can be deployed in the vessel bifurcation using the methods described herein.

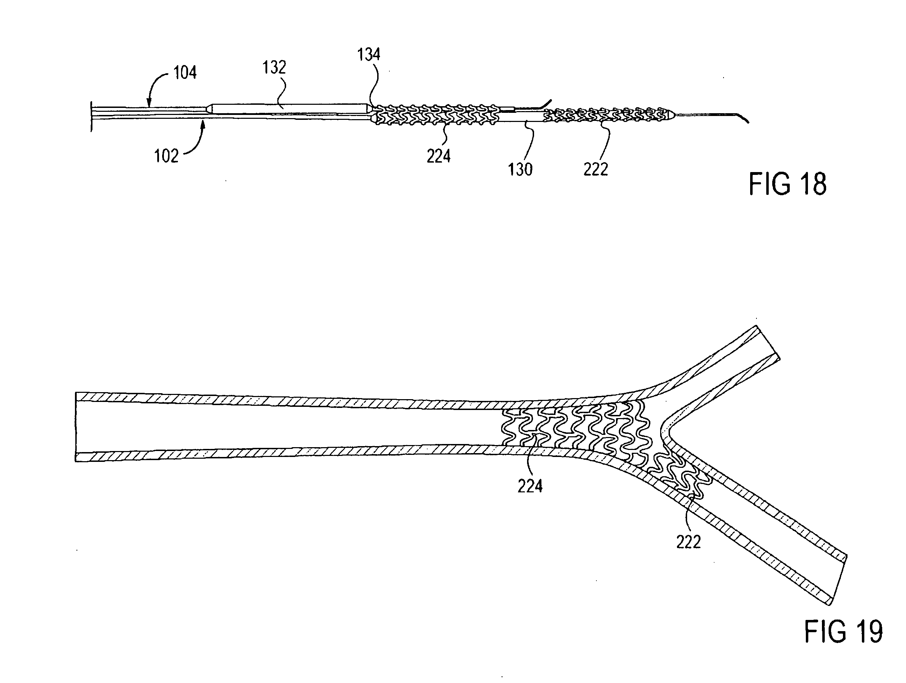

FIG 18 shows a distal portion of a catheter system 100 for stenting bifurcated vessels. The catheter system 100 is similar to that shown in FIG 5 with a first balloon catheter 102 having a first inflatable balloon 130 and a second balloon catheter 104 having a second inflatable balloon 132 and a flexible tubular extension 134 extending distally from the balloon 132. The first and second inflatable balloons 130, 132 are assembled together in a staggered or tandem initial position as shown to provide a low crossing profile. The catheter system 100 can use any of the linking devices 160 described herein to maintain the longitudinal alignment of the catheters 102, 104 during insertion. A distal stent 222 is mounted on a distal portion of the first inflatable balloon 130 and a proximal stent 224 is mounted on a proximal portion of the first inflatable balloon 130 and the flexible tubular extension 134 of the second balloon catheter 104. Preferably, only a small space is left between the distal and proximal stents 222, 224. The distal stent 222 is configured to fit the distal main branch diameter and proximal stent 224 is configured to fit the proximal main branch diameter and the bifurcation itself. Preferably, the proximal stent 224 is configured so that it can be overdilated if necessary to fit the vessel at the bifurcation. In addition, the catheter system 100 may optionally utilize a sidebranch stent 178 mounted on the second balloon 132, as illustrated in FIG 5.

The distal stent 222 and the proximal stent 224 are deployed using sequential and/or simultaneous inflation of the first and second inflatable balloons 130, 132 using the methods described herein. FIG 19 shows a bifurcated vessel after stenting with the catheter system 100 of FIG 18. Using separate distal and proximal stents 222, 224 allows the stents to be independently sized to fit the target vessel and it allows independent expansion of the two stents without any links between them that could cause distortion of one or both stents during deployment.

While the present invention has been described herein with respect to the exemplary embodiments and the best mode for practicing the invention, it will be apparent to one of ordinary skill in the art that many modifications, improvements and subcombinations of the various embodiments, adaptations and variations can be made to the invention without departing from the scope thereof. Although the present invention has been primarily described in relation to angioplasty and stenting of bifurcated blood vessels, the apparatus and methods of the invention can also be used for other applications as well. For example, the catheter system can be used for stenting bifurcated lumens in other organ systems of the body. In addition, the linking devices described herein can be used in other applications where it is desired to hold two or more catheters or similar devices arranged in a side-by-side configuration and aligned with one another along a longitudinal axis. The principles of the invention can also be applied to catheters other than balloon catheters.

## Claims

1. A catheter system comprising:
a first catheter (102) having a shaft with a proximal end and a distal end;
a second catheter (104) having a shaft with a proximal end and a distal end and a linking device (160) **characterized in that** said linking device (160) is attachable near the proximal ends of the catheters for releasably linking the first catheter (102) and the second catheter (104) together in a side-by-side configuration and with the first catheter (102) and the second catheter (104) aligned with one another along a longitudinal axis.

2. The catheter system of claim 1, wherein the first catheter (102) and the second catheter (102) are balloon catheters.

3. The catheter system of claim 2, further comprising a stent (150, 170, 222) mounted on at least one of the balloon catheters.

4. The catheter system of claim 2 or 3, wherein the first catheter (102) and the second catheter (104) are configured as rapid exchange balloon dilatation catheters.

5. The catheter system of claim 4, wherein a proximal section of each rapid exchange balloon dilatation catheter is constructed of hypodermic tubing joined to a flexible distal section.

6. The catheter system of claim 4, wherein at least one (104) of the catheters comprises an elongated flexible extension tube (134) extending distally from the dilatation balloon.

7. The catheter system as claimed in any of claims 1 to 6, wherein the linking device (160) is configured to hold at least one guidewire (140, 142) stationary with respect to the catheter system.

8. The catheter system of claim 1, wherein the first catheter (102) and the second catheter (104) are configured as over-the-wire catheter balloon dilatation catheters.

9. The catheter system as claimed in any of claims 1 to 8, wherein the linking (160) device comprises a body (162) with a first channel (164) and a second channel (166) arranged in a side-by-side configuration, the first channel (164) being configured to releasably hold the shaft of the first catheter (102) and the second channel (166) being configured to releasably hold the shaft of the second catheter (104).

10. The catheter system as claimed in any of claims 1 to 8, wherein the linking device comprises a body (162) with a first channel (164) and a second channel (166) arranged in a side-by-side configuration, a first locking device (180) associated with the first channel (164) configured to releasably hold the shaft of the first catheter (102), and a second locking device (182) associated with the second channel (166) configured to releasably hold the shaft of the second catheter (104).

11. The catheter system as claimed in any of claims 1 to 8, wherein the linking device (160) comprises a first linking member (184) attached to the shaft of the first catheter (102) and a second linking member (186) attached to the shaft of the second catheter (104), the first linking member and the second linking member have interlocking features so that the first linking member (184) and the second linking member (186) can be releasably attached to one another.

12. The catheter system as claimed in any of claims 1 to 8, wherein the linking device comprises a peel-away sheath (190) releasably attaching the shaft of the first catheter (102) and the shaft of the second catheter (104) together.

13. The catheter system as claimed in any of claims 1 to 8, wherein the linking device (160) comprises a split-tube (200) releasably attaching the shaft of the first catheter (102) and the shaft of the second catheter (104) together.

14. The catheter system of claim 5, wherein the linking device (160) comprises a split-tube (200) releasably attaching the shaft of the first catheter (102) and the shaft of the second catheter (104) together, and wherein said split-tube (200) comprises a distal pull-tab (210) to facilitate removal of the linking device (160) from the shafts of the first catheter (102) and the second catheter (104).

15. The catheter system of claim 14, wherein the split-tube (200) of the linking device (160) has a longitudinal split (202) along one side of the split-tube (200) and the distal pull-tab (210) is located on a side of the split-tube (200) opposite to the longitudinal split (202).

16. The catheter system of claim 14, wherein the first catheter (102) and the second catheter (104) are each configured with a proximal hub (122, 124) located at the proximal end of the shaft and a proximal guidewire port (114, 116) located on the shaft distal to the proximal section, and wherein the split-tube (200) of the linking device (160) extends along most of the proximal sections (106, 108) of the first catheter (102) and the second catheter (104) between the proximal hubs (122, 124) and the proximal guidewire ports (114, 116) of the catheters.

17. The catheter system of claim 14, wherein the split-tube (200) of the linking device (160) has an inner lumen (204) that is sized and configured to hold the proximal sections (106, 108) of the first catheter (102) and the second catheter (104) together with sufficient friction that the catheter system can be advanced as a unit without any relative longitudinal movement between the first catheter (102) and the second catheter (104).

18. The catheter system of claim 14, wherein the split-tube (200) of the linking device (160) has a first inner lumen (206) that is sized and configured to hold the proximal section (106) of the first catheter (102) and a second inner lumen (208) that is sized and configured to hold the proximal section (108) of the second catheter (104), said first and second inner lumens (206, 208) being configured with sufficient friction that the catheter system can be advanced as a unit without any relative longitudinal movement between the first catheter (102) and the second catheter (104).

## Patentansprüche

1. Kathetersystem, umfassend:
einen ersten Katheter (102), der einen Schaft mit einem proximalen Ende und einem distalen Ende hat,
einen zweiten Katheter (104), der einen Schaft mit einem proximalen Ende und einem distalen Ende hat, und
eine Verbindungseinrichtung (160),
**dadurch gekennzeichnet, daß** die Verbindungseinrichtung (160) nahe den proximalen Enden der Katheter befestigt werden kann, um den ersten Katheter (102) und den zweiten Katheter (104) in einer Seite-an-Seite-Konfiguration, wobei der erste Katheter (102) und der zweite Katheter (104) längs einer Längsachse miteinander ausgerichtet sind, lösbar miteinander zu verbinden.

2. Kathetersystem nach Anspruch 1, wobei der erste Katheter (102) und der zweite Katheter (104) Ballonkatheter sind.

3. Kathetersystem nach Anspruch 2, das ferner einen Stent (150, 170) umfaßt, der an wenigstens einem der Ballonkatheter angebracht ist.

4. Kathetersystem nach Anspruch 2 oder 3, wobei der erste Katheter (102) und der zweite Katheter (104) als Schnellwechsel-Ballondilatationskatheter konfiguriert sind.

5. Kathetersystem nach Anspruch 4, wobei eine proximale Sektion jedes Schnellwechsel-Ballondilatationskatheters aus Subkutanschlauch aufgebaut ist, der mit einer flexiblen distalen Abschnitt verbunden ist.

6. Kathetersystem nach Anspruch 4, wobei wenigstens einer (104) der Katheter eine längliche flexible Verlängerungsröhre (134) umfaßt, die sich in Distalrichtung von dem Dilatationsballon aus erstreckt.

7. Kathetersystem nach einem der Ansprüche 1 bis 6, wobei die Verbindungseinrichtung (160) dazu konfiguriert ist, wenigstens einen Führungsdraht (140, 142) unbeweglich in Bezug zu dem Kathetersystem zu halten.

8. Kathetersystem nach Anspruch 1, wobei der erste Katheter (102) und der zweite Katheter (104) als Over-the-Wire-Ballondilatationskatheter konfiguriert sind.

9. Kathetersystem nach einem der Ansprüche 1 bis 8, wobei die Verbindungseinrichtung (160) einen Korpus (162) mit einem ersten Kanal (164) und einem zweiten Kanal (166), die in einer Seite-an-Seite-Konfiguration angeordnet sind, umfaßt, wobei der erste Kanal (164) dazu konfiguriert ist, den Schaft des ersten Katheter (102) lösbar zu halten, und der zweite Kanal (166) dazu konfiguriert ist, den Schaft des zweiten Katheters (104) lösbar zu halten.

10. Kathetersystem nach einem der Ansprüche 1 bis 8, wobei die Verbindungseinrichtung einen Korpus (162) mit einem ersten Kanal (164) und einem zweiten Kanal (166), die in einer Seite-an-Seite-Konfiguration angeordnet sind, eine erste Arretierungseinrichtung (180), die mit dem ersten Kanal (164) verknüpft und dazu konfiguriert ist, den Schaft des ersten Katheter (102) lösbar zu halten, und eine zweite Arretierungseinrichtung (182), die mit dem zweiten Kanal (166) verknüpft und dazu konfiguriert ist, den Schaft des zweiten Katheters (104) lösbar zu halten, umfaßt.

11. Kathetersystem nach einem der Ansprüche 1 bis 8, wobei die Verbindungseinrichtung (160) ein erstes Verbindungselement (184), das an dem Schaft des ersten Katheters (102) befestigt ist, und ein zweites Verbindungselement (186), das an dem Schaft des zweiten Katheters (104) befestigt ist, umfaßt, wobei das erste Verbindungselement und das zweite Verbindungselement ineinandergreifende Merkmale haben, so daß das erste Verbindungselement (184) und das zweite Verbindungselement (186) lösbar aneinander befestigt werden können.

12. Kathetersystem nach einem der Ansprüche 1 bis 8, wobei die Verbindungseinrichtung eine Abziehhülle (190) umfaßt, die den Schaft des ersten Katheters (102) und den Schaft des zweiten Katheters (104) lösbar aneinander befestigt.

13. Kathetersystem nach einem der Ansprüche 1 bis 8, wobei die Verbindungseinrichtung (160) einen Schlitztubus (200) umfaßt, die den Schaft des ersten Katheters (102) und den Schaft des zweiten Katheters (104) lösbar aneinander befestigt.

14. Kathetersystem nach Anspruch 5, wobei die Verbindungseinrichtung (160) einen Schlitztubus (200) umfaßt, die den Schaft des ersten Katheters (102) und den Schaft des zweiten Katheters (104) lösbar aneinander befestigt und wobei der Schlitztubus (200) eine distale Zuglasche (210) umfaßt, um ein Entfernen der Verbindungseinrichtung (160) von den Schäften des ersten Katheters (102) und des zweiten Katheters (104) zu erleichtern.

15. Kathetersystem nach Anspruch 14, wobei der Schlitztubus (200) der Verbindungseinrichtung (160) einen Längsschlitz (202) längs einer Seite des Schlitztubus (200) hat und die distale Zuglasche (210) auf einer dem Längsschlitz (202) gegenüberliegenden Seite des Schlitztubus (200) angeordnet ist.

16. Kathetersystem nach Anspruch 14, wobei der erste Katheter (102) und der zweite Katheter (104) jeder mit einem proximalen Verbindungstück (122, 124), das an dem proximalen Ende des Schafts angeordnet ist, und einer proximalen Führungsdraht-Öffnung (114, 116), die an dem Schaft, distal zu der proximalen Sektion, angeordnet ist, konfiguriert ist und wobei sich der Schlitztubus (200) der Verbindungseinrichtung (160) längs des Großteils der proximalen Sektionen (106, 108) des ersten Katheters (102) und des zweiten Katheters (104) zwischen den proximalen Verbindungstücken (122, 124) und den proximalen Führungsdraht-Öffnungen (114, 116) der Katheter erstreckt.

17. Kathetersystem nach Anspruch 14, wobei der Schlitztubus (200) der Verbindungseinrichtung (160) ein inneres Lumen (204) hat, das dafür bemessen und konfiguriert ist, die proximalen Sektionen (106, 108) des ersten Katheters (102) und des zweiten Katheters (104) mit ausreichender Reibung zusammenzuhalten, daß das Kathetersystem als eine Einheit ohne jegliche relative Längsbewegung zwischen dem ersten Katheter (102) und dem zweiten Katheter (104) vorgeschoben werden kann.

18. Kathetersystem nach Anspruch 14, wobei der Schlitztubus (200) der Verbindungseinrichtung (160) ein erstes inneres Lumen (206), das dafür bemessen und konfiguriert ist, die proximale Sektion (106) des ersten Katheters (102) zu halten, und ein zweites inneres Lumen (208) besitzt, das dafür bemessen und konfiguriert ist, die proximale Sektion (108) des zweiten Katheters (104) zu halten, wobei das erste und das zweite innere Lumen (206, 208) mit ausreichender Reibung konfiguriert sind, daß das Kathetersystem als eine Einheit ohne jegliche relative Längsbewegung zwischen dem ersten Katheter (102) und dem zweiten Katheter (104) vorgeschoben werden kann.

## Revendications

1. Système de cathéter, comprenant :
un premier cathéter (102) ayant une gaine avec une extrémité proximale et une extrémité distale
un second cathéter (104) ayant une gaine avec une extrémité proximale et une extrémité distale, et un dispositif de liaison (160) ;
**caractérisé en ce que** ledit dispositif de liaison (160) est susceptible d'être attaché à proximité des extrémités proximales des cathéters pour relier de façon détachable le premier cathéter (102) et le second cathéter (104) ensemble dans une configuration côte à côte et avec le premier cathéter (102) et le second cathéter (104) alignés l'un à l'autre le long d'un axe longitudinal.

2. Système de cathéter selon la revendication 1, dans lequel le premier cathéter (102) et le second cathéter (102) sont des cathéters à ballon.

3. Système de cathéter selon la revendication 2, comprenant en outre un stent (150, 170, 222) monté sur l'un au moins des cathéters à ballon.

4. Système de cathéter selon la revendication 2 ou 3, dans lequel le premier cathéter (102) et le second cathéter (104) sont configurés comme des cathéters de dilatation à ballon à échange rapide.

5. Système de cathéter selon la revendication 4, dans lequel une section proximale de chaque cathéter de dilatation à ballon à rechange rapide est construite d'un tubage hypodermique réuni à une section distale flexible.

6. Système de cathéter selon la revendication 4, dans lequel l'un au moins (104) des cathéters comprend un tube d'extension flexible allongé (134) s'étendant en direction distale depuis le ballon de dilatation.

7. Système de cathéter selon l'une quelconque des revendications 1 à 6, dans lequel le dispositif de liaison (160) est configuré pour tenir au moins un fil-guide (140, 142) stationnaire par rapport au système de cathéter.

8. Système de cathéter selon la revendication 1, dans lequel le premier cathéter (102) et le second cathéter (104) sont configurés comme des cathéters de dilatation avec ballon guidé sur fil ("over-the-wire").

9. Système de cathéter selon l'une quelconque des revendications 1 à 8, dans lequel le dispositif de liaison (160) comprend un corps (162) avec un premier canal (164) et un second canal (166) agencés dans une configuration côte à côte, le premier canal (164) étant configuré pour tenir de manière libérable la gaine du premier cathéter (102) et le second canal (166) étant configuré pour tenir de manière libérable la gaine du second cathéter (104).

10. Système de cathéter selon l'une quelconque des revendications 1 à 8, dans lequel le dispositif de liaison comprend un corps (162) avec un premier canal (164) et un second canal (166) agencés dans une configuration côte à côte, un premier dispositif de blocage (180) associé au premier canal (164) configuré pour tenir de manière libérable la gaine du premier cathéter (102), et un second dispositif de blocage (182) associé au second canal (166) configuré pour tenir de manière libérable la gaine du second cathéter (104).

11. Système de cathéter selon l'une quelconque des revendications 1 à 8, dans lequel le dispositif de liaison (160) comprend un premier élément de liaison (184) attaché à la gaine du premier cathéter (102) et un second élément de liaison (186) attaché à la gaine du second cathéter (104), le premier élément de liaison et le second élément de liaison ayant des caractéristiques d'interverrouillage de telle sorte que le premier élément de liaison (184) et le second élément de liaison (186) peuvent être attachés de manière libérable l'un à l'autre.

12. Système de cathéter selon l'une quelconque des revendications 1 à 8, dans lequel le dispositif de liaison comprend une gaine pelable (190) qui attache de manière libérable la gaine du premier cathéter (102) et la gaine du second cathéter (104) ensemble.

13. Système de cathéter selon l'une quelconque des revendications 1 à 8, dans lequel le dispositif de liaison (160) comprend un tube fendu (200) qui attache de manière libérable la gaine du premier cathéter (102) et la gaine du second cathéter (104) ensemble.

14. Système de cathéter selon la revendication 5, dans lequel le dispositif de liaison (160) comprend un tube fendu (200) qui attache de manière libérable la gaine du premier cathéter (102) et la gaine du second cathéter (104) ensemble, et dans lequel ledit tube fendu (200) comprend une languette à tirer (210) distale pour faciliter l'enlèvement du dispositif de liaison (160) depuis les gaines du premier cathéter (102) et du second cathéter (104).

15. Système de cathéter selon la revendication 14, dans lequel le tube fendu (200) du dispositif de liaison (160) comporte une fente longitudinale (202) le long d'un côté du tube fendu (200) et la languette à tirer (210) distale située sur un côté du tube fendu (200) à l'opposé de la fente longitudinale (202).

16. Système de cathéter selon la revendication 14, dans lequel le premier cathéter (102) et le second cathéter (104) sont configurés chacun avec un raccord proximal (122, 124) situé à l'extrémité proximale de la gaine et un orifice de fil-guide proximal (114, 116) situé sur la gaine en situation distale par rapport à la section proximale, et dans lequel le tube fendu (200) et le dispositif de liaison (160) s'étendent le long d'une majeure partie des sections proximales (106, 108) du premier cathéter (102) et du second cathéter (104) entre les raccords proximaux (122, 124) et les orifices de fil-guide proximaux (114, 116) des cathéters.

17. Système de cathéter selon la revendication 14, dans lequel le tube fendu (200) du dispositif de liaison (160) comporte un lumen intérieur (204) avec une taille et une configuration propres à tenir les sections proximales (106, 108) du premier cathéter (102) et du second cathéter (104) ensemble avec une friction suffisante de telle sorte que le système de cathéter peut être avance comme une unité sans aucun mouvement longitudinal relatif entre le premier cathéter (102) et le second cathéter (104).

18. Système de cathéter selon la revendication 14, dans lequel le tube fendu (200) du dispositif de liaison (160) comporte un premier lumen intérieur (206) avec une taille et une configuration propres à tenir la section proximale (106) du premier cathéter (102) et un second lumen intérieur (208) avec une taille et une configuration propres à tenir la section proximale (108) du second cathéter (104), ledit premier et ledit second lumen intérieur (206, 208) étant configurés avec une friction suffisante pour que le système de cathéter puisse être avancé comme une unité sans aucun mouvement longitudinal relatif entre le premier cathéter (102) et le second cathéter (104).
